# EUROPEAN PATENT SPECIFICATION

(11) **EP 2 378 979 B1**
(45) Date of publication and mention of the grant of the patent: **10.05.2017**
(21) Application number: 09760568.7
(22) Date of filing: 18.11.2009
(51) Int. Cl.: A61B 10/00

(54) **DEVICE FOR COLLECTING FIRST PASS URINE**
VORRICHTUNG ZUR SAMMLUNG DES ERSTEN URINS
DISPOSITIF DE COLLECTE D'URINE DE DÉBUT DE MICTION

(30) Priority: 18.11.2008 GB 0821057
(43) Date of publication of application: 26.10.2011
(73) Proprietor: Knight Scientific Limited, Devon PL4 7AG (GB)
(72) Inventor: KNIGHT, Jan, Devon PL4 7AG (GB); KNIGHT, Robert, Devon PL4 7AG (GB)
(74) Representative: Wilkinson, Stephen John
(86) International application number: PCT/GB2009/051561
(87) International publication number: WO 2010/058210

(56) References cited:
- WO-A1-2005/107602
- US-A- 3 982 898
- US-A- 4 494 581
- US-A- 5 069 878
- US-A- 5 904 677
- US-A- 6 024 709
- US-A1- 2004 267 159
- US-A1- 2005 177 070
- US-B1- 6 210 909

## Description

The invention relates to a device for collecting a sample of first pass urine from a donor. The invention is defined by independent claim 1. Preferred embodiments are as defined by the dependent claims. It is frequently necessary, as part of the diagnosis of a wide range of diseases, to subject a sample of urine taken from a patient to medical testing and analysis. Typically, a sample of urine will be analysed to determine the concentrations of various chemical substances in the urine and/or to identify the presence of certain substances not normally present in the urine of a healthy individual.

For most procedures, medical testing requires a sample of urine which is free from contaminants. Possible contaminants might, for instance, be bacteria from the urogenitary tract or bladder or kidney cells. The midstream portion of urine, which is urine from the middle of the bladder, is considered to be best for this purpose. If a midstream portion is required for medical testing, it is generally necessary to ensure that this does not become contaminated during the collection procedure. Devices which are specifically designed to collect midstream urine for medical testing typically involve means for ensuring that a sample of midstream urine is isolated without it having been contaminated by contact with the prior voided first pass urine. Such devices may, for instance, contain means for diverting the flow of urine entering the device from a first container, into which first pass and midstream urine enters, to a second container, into which only subsequently voided, uncontaminated midstream urine is passed. Typically, the collected mixture of first pass and midstream urine would, then, be disposed of. US 4,494,581 discloses a device for isolating a forestream sample of urine from a subsequently collected midstream specimen. In this device, a urine-absorbent material which swells to many times its volume when wetted is disposed in a forestream receiving chamber and a moveable valve member is disposed above the urine-absorbent material. As urine enters the forestream receiving chamber and is absorbed by the urine absorbent material, this material swells and, as it does so, forces the valve member against the inflow opening for the chamber to block further entry of liquid into the chamber.

There are, however, circumstances in which the first pass urine needs to be subjected to testing or analysis. For instance, the first pass urine of a man infected with the bacterium, *Chlamydia trachomatis,* contains detectable species which are not present, or are only detectable with difficulty, in midstream portions of urine. If not detected and treated early, Chlamydia infections in both sexes can lead to serious, long term problems. To enable urine testing, for instance, for the presence of sexually transmitted diseases, it is necessary to use a sample of first pass urine which is not diluted by midstream urine.

It is an object of the present invention to provide an apparatus which enables the collection of first pass urine not diluted by midstream urine.

Accordingly, the present invention provides a device for collecting a sample of the first pass portion of urine voided by a donor isolated from the midstream portion of the urine which device comprises a first part comprising an opening at its upper end adapted to receive urine voided by a donor, a urine collection body downstream of the upper end and a closable exit located downstream of the urine collection body and at the lower end of the first part, the exit providing a circular orifice, a second part located downstream of the exit of the first part and comprising a collecting vessel which is in communication with the exit of the first part wherein the circular orifice provided by the exit of the first part defines an inlet into the collecting vessel and has a diameter which is smaller than the diameter of the collecting vessel at the upper end thereof, wherein the collecting vessel is in the form of a container having a closed end which container contains a spherical bead having a diameter which is greater than the diameter of the circular orifice for blocking the inlet into the collecting vessel, characterised in that the spherical bead is freely moveable within the container and is configured to float in urine such that, in use, it blocks the inlet into the collecting vessel on the filling of the collecting vessel with urine so as to cause further voided urine to be collected in the urine collection body of the first part.

According to a preferred embodiment, the first part of the device of the present invention comprises a funnel having, at or near to its narrower end, a closable exit. The funnel may be provided with an additional, openable and closable outlet means located above its narrower end to facilitate removal of urine collected in the urine collection body of the funnel after the first pass urine has been collected in the second part of the device.

According to another preferred embodiment, the second part of the device of the invention is capable of being detached from the first part of the device. Such detachability enables the second part of the device, which after use will contain first pass urine, to be separated from the remaining parts of the device and capped or closed by a cap or lid so that the urine sample can be stored or moved without suffering contamination or spillage.

The collecting vessel of the second part of the device contains a spherical bead having a diameter which is greater than the diameter of the circular orifice at the exit of the first part of the device. The spherical bead is capable of floating in urine such that it rises with the rising level of first pass urine in the collecting vessel until it blocks the entry into the collecting vessel, at the inlet, of further urine. Such further urine, after closure of the inlet into the second part of the device, then collects in the collection body of the first part of the device. The spherical bead has a diameter greater than the diameter of the inlet defined by the circular orifice provided by the exit of the first part of the device such that it closes the inlet when the level of first pass urine reaches its maximum possible level in the collecting vessel of the second part of the device.

Preferably, either or both of the first part and the second part of the device will be formed of a plastics material. Since the parts of the device are intended to be disposed of after use, the manufacturing cost of the device needs to be low.

According to a further embodiment of the invention, a filter is provided in the device located such that first pass urine voided into the device will pass through it. The filter will comprise material capable of filtering one or more substances or matter from the urine, for example cellular matter, which may be captured by the filter for subsequent analysis. Preferably, the filter material is capable of removing leucocytes from the first pass urine. Typically, the filter will be located at or near to the lower end of the first part of the device to ensure that the first pass urine passes through the filter.

According to a yet further embodiment, the device also includes a stand to support the first and second parts described above.

In a particularly preferred embodiment, the first and second parts of the device are housed in a rigid housing which is adapted to sit stably on a flat, horizontal surface while maintaining the first and second parts in a typically upright position such that the device is positioned ready for use by a patient. The housing may, for instance, be selected from a box, tube, jar, bottle or flask. The base of the housing must, of course, be capable of stably supporting the urine collection parts of the device in an appropriate position such that these do not tip over during or after use. Preferably, a funnel which, according to this embodiment, forms the urine collection body of the first part is formed integrally with the walls of the housing. This may, in particular, be achieved simply, when the housing and the funnel are formed from a suitable plastics material, by a moulding process. Access to the collecting vessel of the second part may be achieved by an opening provided in a side wall of the housing such that, in use, the contents of the collecting vessel may be removed from the collecting vessel through the opening in the side wall of the housing. For instance, the collecting vessel may be connected to the side wall of the housing at the opening by a tubular member and wherein the wall of the collecting vessel separating the collecting vessel from the interior of the tubular member, or a portion thereof, comprises a pierceable membrane. The tubular member may be formed integrally with the wall of the collecting vessel and/or may be formed integrally with the side wall of the housing.

According to a further preferred embodiment, the housing has a closable open top. The closable open top of the housing may, for instance, be provided with a lid or cap. In an especially preferred embodiment, the open top of the housing is closable by a lid which has, on its internal surface, a downwardly projecting stopper which, when the lid is closed, stops or plugs the exit provided at the lower end of the first part of the device. The lid may also be provided with means enabling access to urine collected in the urine collection body of the first part of the device. For instance, the lid may be provided with a portion formed by a pierceable membrane, such that a sample of the urine collected in the first part of the device may be removed by means of a syringe fitted with a needle or using a vacuum tube. Alternatively, the lid may be formed to have a permanent hole which is closable by a resealable cap. In the case when the lid is formed with a permanent hole, the hole may be formed to enable the contents of the first part to be poured easily through the hole such as by shaping the edges of the hole to provide a spout to facilitate pouring.

In order that the invention can be fully understood and readily carried into effect, the same will now be described by way of example only, with reference to the accompanying drawings, of which:-
Figure 1 is a cross-sectional view of an embodiment of the device of the invention;
Figure 2 is a cross-sectional view of the device shown in Figure 1 in use;
Figure 3 is a perspective view of a different device embodying the invention;
Figure 4 is an exploded view of the device illustrated in Figure 3 showing the component parts of the device.

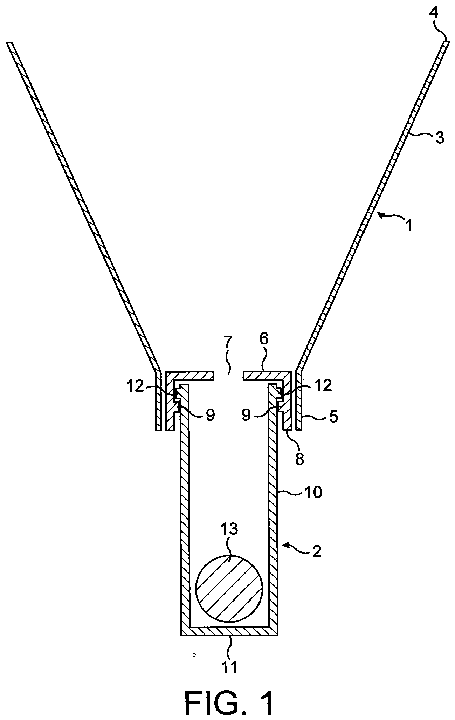

The urine collection device, as shown in the drawings, is used to collect a first pass urine sample for analysis/medical testing. As shown in Figure 1, the device comprises a first part 1 and a second part 2. The first part 1 comprises a urine collection body in the form of a funnel 3 having a larger end 4 and a narrower end 5. The funnel may be of any suitable size, shape or dimension. It may also be provided with a gulley to direct urine flow to the narrower end. The larger, upper end 4 of the funnel provides a receiving end for placing adjacent a donor to collect urine. Fitted within the narrower end 5 of the funnel is an exit element 6 containing an orifice 7 defining an outlet into the second part 2. The exit element 6 has a side wall 8 which forms a seal with the internal surface of the narrower end of the funnel. The internal surface of side wall 8 is provided with a flange 9. If desired, a filter material (not shown) capable of removing matter, for example leucocytes, from urine passing through it may be provided in the funnel 3. Typically, such a filter material, if used, will be located at or near to the narrower end 5 of the funnel 3 to ensure that urine to be collected will pass through it. Preferably, the filter material will be located immediately above and/or within orifice 7. The second part 2 of the device, which is located downstream of the exit element 6 of the first part, comprises a collecting vessel in the form of a container 10 having a closed end 11. Prior to use of the device of the invention, a container having the desired capacity will be selected depending on the volume of the sample of first pass urine required. The container 10 comprises a tube at its uppermost end which is in communication with the exit element 6. The external surface of container 10 adjacent its upper end is provided with a screw thread 12 which engages with the flange 9 provided on the internal surface of the side wall 8 of the exit element 6. The container 10 contains a spherical bead 13 which is freely moveable within the container and which is buoyant in urine. The device may be held in a stable arrangement adjacent the donor, during use, by means of a support (not shown).

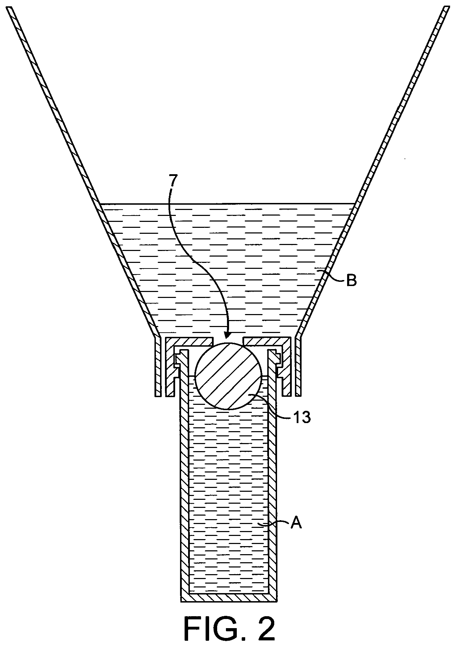

In use, the donor places the device of the invention in an appropriate position so that urine may be voided into the funnel 3. The voided urine flows into the funnel 3 and passes through orifice 7 of exit element 6 into the container 10. As the level of urine in container 10 rises, the buoyant spherical bead 13 floats at the surface of the urine in the container and so rises as the level of the urine rises in the container. When the level of first pass urine (A) in the container reaches its maximum, the spherical bead 13 is pushed upwards against the orifice 7 so as to close the orifice to prevent further urine passing through into the container 10, as shown in Figure 2. Further urine (B) voided by the donor is then collected in the body of the funnel.

When the donor has finished voiding urine, the urine collected in the body of the funnel may be tipped into a suitable waste outlet or into another container, if it is to be analysed. Tipping of the funnel to remove the urine contained in the body of the funnel can be achieved without loss of the first pass urine in the container 10. The container, containing a sample of first pass urine, may then be detached from the first part of the device by unscrewing it from the flange 9 and then fitting with a suitable cap or lid to prevent contamination or spillage of the contents of the container. Alternatively, when the urine contained in the body of the funnel has been removed by tipping, the urine contained in the part 2 may be poured via part 1 into a suitable container by rotating the device so that part 2 is raised above the horizontal such that the bead 13 will float to the closed end of the part 2 allowing the contained urine to flow out.

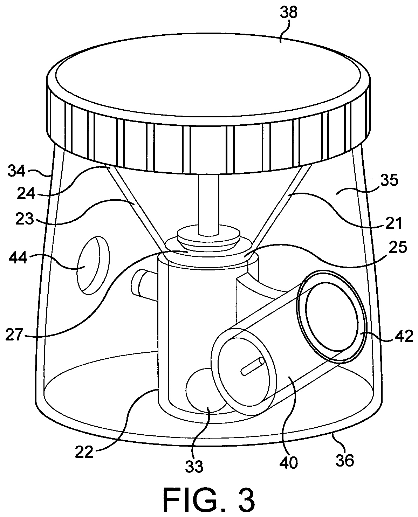

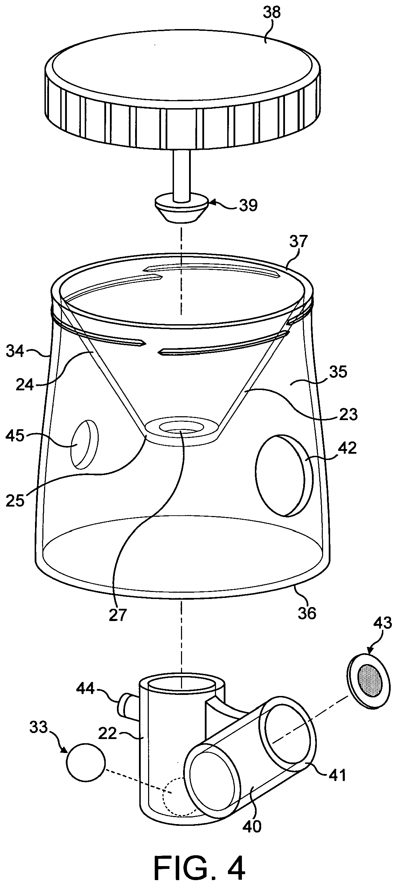

Turning now to Figure 3, in a different device embodying the invention, the first part 21 comprises a funnel 23 formed of a moulded plastics material which, at its larger end 24, is formed integrally with the walls 34 of housing 35 similarly formed from moulded plastics material. The housing 35 has an open bottom 36 which provides a stable seat for the device. The top 37 of the housing 35, better seen in the exploded view shown in Figure 4, is closable by a lid 38. The funnel 23 has, at its narrow end 25, an orifice 27. The lid 38 has, projecting from its inner surface, a stopper 39. When the lid is closed over the top of the housing, the stopper 39 plugs the orifice 27 at the bottom end 25 of the funnel. The second part 22 of the device comprises a collecting vessel which, when the device is in use, is located immediately beneath the bottom end of the funnel so that first pass urine may flow through the orifice into the vessel. The collecting vessel contains a float seal 33 which, in use, floats in the urine collecting in the vessel and which, when the level of urine in the vessel rises to a maximum, closes the orifice 27 such that further urine voided by the patient is then collected in the funnel and does not mix with the first pass urine in the vessel. The collecting vessel 22 is formed integrally with a tubular member 40 having an end 41 remote from the collecting vessel which is aligned with an opening 42 provided in the side wall of the housing. The opening 42 in the housing wall will typically be closed when not in use, for instance by a cap, lid or bung. The interior of the collecting vessel 22 is separated from the interior of the tubular member 40 by a pierceable membrane 43. The contents of the collecting vessel may, thus, be removed via the tubular member using a syringe having a hollow needle which pierces the membrane 43. A valve 44 is now provided in the side wall of the collecting vessel 22 for allowing air into or out of the collecting vessel. The valve can be accessed through a second opening 45 in the wall of the housing.

## Claims

1. A device for collecting a sample of the first pass portion of urine voided by a donor isolated from the midstream portion of the urine which device comprises a first part (1) comprising an opening at its upper end (4) adapted to receive urine voided by a donor, a urine collection body (3) downstream of the upper end and a closable exit (6) located downstream of the urine collection body and at the lower end (5) of the first part (1), the exit (6) providing a circular orifice (7), a second part (2) located downstream of the exit (6) of the first part (1) and comprising a collecting vessel which is in communication with the exit (6) of the first part (1) wherein the circular orifice (7) provided by the exit (6) of the first part (1) defines an inlet into the collecting vessel and has a diameter which is smaller than the diameter of the collecting vessel at the upper end thereof, wherein the collecting vessel is in the form of a container (10) having a closed end (11) which container contains a spherical bead (13) having a diameter which is greater than the diameter of the circular orifice (7) for blocking the inlet into the collecting vessel, **characterised in that** the spherical bead (13) is freely moveable within the container (10) and is capable of floating in urine such that, in use, it blocks the inlet into the collecting vessel on the filling of the collecting vessel with urine so as to cause further voided urine to be collected in the urine collection body of the first part and further such that, in use, the bead (13) is capable of floating to the closed end of the container (10) when the container (10) is rotated.

2. A device according to claim 1, wherein the first part (1) comprises a funnel (3) having a closable exit (6) provided at or near to its narrower end (5).

3. A device according to claim 2 which is housed in a rigid housing (35) having a closable open top (37), the housing having a bottom (36) adapted such that the device is capable of sitting stably on a flat, horizontal surface.

4. A device according to claim 3, wherein the funnel (3) of the first part (1) is formed integrally with the walls (34) of the housing.

5. A device according to either claim 3 or claim 4, wherein access to the collecting vessel in the second part (2) is provided by an opening (42) in a side wall of the housing (35) such that, in use, the contents of the collecting vessel may be removed from the collecting vessel through the opening (42) in the side wall of the housing (35).

6. A device according to claim 5, wherein the collecting vessel is connected to the side wall of the housing (35) at the opening (42) therein by a tube (40) and wherein the wall of the collecting vessel separating the collecting vessel from the interior of the tube comprises a pierceable membrane (43).

7. A device according to claim 6, wherein the tube (40) is integrally formed with the wall of the collecting vessel.

8. A device according to either claim 6 or claim 7, wherein the tube (40) is integrally formed with the side wall of the housing (35).

9. A device according to any one of claims 6 to 8, wherein the opening (42) in the side wall of the housing (35) is closed by an openable closure member (44).

10. A device according to any one of claims 3 to 9, wherein the closable open top (37) of the housing (35) is provided with a lid (38).

11. A device according to claim 10, wherein the lid (38), on its internal surface, has a downwardly projecting stopper (39) whereby closure of the lid (38) on the top (37) of the housing (35) causes the stopper (39) to plug the exit (27) provided at the lower end of the first part.

12. A device according to either claim 10 or claim 11, wherein the lid (38) comprises a section formed by a pierceable membrane.

13. A device according to either claim 10 or claim 11, wherein the lid (38) has a sealable opening therein.

14. A device according to claim 13, wherein the sealable opening in the lid (38) is formed to provide a spout.

15. A device according to either claim 1 or claim 2, wherein the second part (2) of the device is capable of being detached from the first part of the device (1).

16. A device according to any one of claims 1 to 15, which additionally comprises a filter material at or near the lower end of the first part.

17. A device according to claim 16, wherein the filter material is one that is capable of removing leucocytes from the first pass portion of urine that passes through it.

18. A method of obtaining a sample of first pass urine from a donor which comprises providing a device according to any one of claims 1 to 17 to a donor, the donor voiding urine into the first part of the device, and collecting the sample of first pass urine in the second part of the device.

## Patentansprüche

1. Vorrichtung zum Sammeln einer Probe vom ersten Durchlaufabschnitt ausgeschiedenen Urins eines Spenders, isoliert aus dem Mittelstrahl des Urins, wobei die Vorrichtung einen ersten Teil (1) mit einer Öffnung am oberen Ende (4) zum Aufnehmen des ausgeschiedenen Urins eines Spenders, einen Urinsammelbeutel (3), der sich dem oberen Ende nachgeschaltet befindet, und einen verschließbaren Ausgang (6), der sich dem Urinsammelbeutel und dem unteren Ende (5) des ersten Teils (1) nachgeschaltet befindet, umfasst, wobei der Ausgang (6) eine kreisförmige Öffnung (7), einen zweiten Teil (2), der sich dem Ausgang (6) des ersten Teils (1) nachgeschaltet befindet, bereitstellt und ein Sammelgefäß umfasst, der in Verbindung mit dem Ausgang (6) des ersten Teils (1) steht, wobei die vom Ausgang (6) des ersten Teils (1) bereitgestellte kreisförmige Öffnung (7) einen Zulauf in das Sammelgefäß definiert und einen Durchmesser hat, der kleiner als der Durchmesser des Sammelgefäßes an dessen oberen Ende ist, wobei das Sammelgefäß die Form eines Behälters (10) mit einem geschlossenen Ende (11) aufweist, wobei der Behälter eine kleine Kugel (13) mit einem Durchmesser hat, der größer als der Durchmesser der kreisförmigen Öffnung (7) ist, um den Zulauf in das Sammelgefäß zu blockieren, **dadurch gekennzeichnet, dass** die Kugel (13) im Behälter (10) frei beweglich und in der Lage ist, im Urin zu schwimmen, so dass sie bei Gebrauch den Zulauf zum Sammelgefäß beim Befüllen des Sammelgefäßes mit Urin blockiert, um ein Sammeln von weiterem ausgeschiedenen Urin im Urinsammelbeutel des ersten Teils zu bewirken, und dass ferner die Kugel (13) bei Gebrauch in der Lage ist, zum geschlossen Ende des Behälters (10) zu schwimmen, wenn der Behälter (10) gedreht wird.

2. Vorrichtung nach Anspruch 1, wobei der erste Teil (1) einen Trichter (3) mit einem verschließbaren Ausgang (6) umfasst, der am oder in der Nähe seines schmaleren Endes (5) angeordnet ist.

3. Vorrichtung nach Anspruch 2, die in einem starren Gehäuse (35) mit einem verschließbaren oberen Ende (37) untergebracht ist, wobei das Gehäuse einen Boden (36) hat, der so angepasst ist, dass die Vorrichtung in der Lage ist, stabil auf einer ebenen, horizontalen Oberfläche zu lagern.

4. Vorrichtung nach Anspruch 3, wobei der Trichter (3) des ersten Teils (1) einstückig mit den Wänden (34) des Gehäuses ausgebildet ist.

5. Vorrichtung nach einem der Ansprüche 3 oder 4, wobei der Zugang zum Sammelgefäß des zweiten Teils (2) durch eine Öffnung (42) in einer Seitenwand des Gehäuses (35) so bereitgestellt wird, dass bei Gebrauch der Inhalt des Sammelgefäßes durch die Öffnung (42) in der Seitenwand des Gehäuses aus dem Sammelgefäß entfernt werden kann.

6. Vorrichtung nach Anspruch 5, wobei das Sammelgefäß mit der Seitenwand des Gehäuses (35) an der Öffnung (42) darin durch ein Röhrchen (40) verbunden ist und wobei die Wand des Sammelgefäßes, die das Sammelgefäß vom Inneren des Röhrchens trennt, eine durchstechbare Membran (43) umfasst.

7. Vorrichtung nach Anspruch 6, wobei das Röhrchen (40) einstückig mit der Wand des Sammelgefäßes ausgebildet ist.

8. Vorrichtung nach einem der Ansprüche 6 oder 7, wobei das Röhrchen (40) einstückig mit der Seitenwand des Gehäuses (35) ausgebildet ist.

9. Vorrichtung nach einem der Ansprüche 6 bis 8, wobei die Öffnung (42) in der Seitenwand des Gehäuses (35) durch ein zu öffnendes Verschlussglied (44) verschlossen wird.

10. Vorrichtung nach einem der Ansprüche 3 bis 9, wobei das verschließbare obere Ende (37) des Gehäuses (35) mit einem Deckel (38) versehen ist.

11. Vorrichtung nach Anspruch 10, wobei der Deckel (38) auf seiner Innenseite einen nach unten gerichteten Stöpsel (39) hat, wobei das Schließen des Deckels (38) auf der Oberseite (37) des Gehäuses (35) dafür sorgt, dass der Stöpsel (39) den Ausgang (27) am unteren Ende des ersten Teils verstopft.

12. Vorrichtung nach einem der Ansprüche 10 oder 11, wobei der Deckel (38) einen Abschnitt umfasst, der von einer durchstechbaren Membran ausgebildet ist.

13. Vorrichtung nach einem der Ansprüche 10 oder 11, wobei der Deckel (38) eine verschließbare Öffnung darin aufweist.

14. Vorrichtung nach Anspruch 13, wobei die verschließbare Öffnung im Deckel (38) so geformt ist, dass sie einen Ausguss bildet.

15. Vorrichtung nach einem der Ansprüche 1 oder 2, wobei der zweite Teil (2) der Vorrichtung vom ersten Teil der Vorrichtung (1) gelöst werden kann.

16. Vorrichtung nach einem der Ansprüche 1 bis 15, die zusätzlich ein Filtermaterial an oder in der Nähe der unteren Endes des ersten Teils umfasst.

17. Vorrichtung nach Anspruch 16, wobei das Filtermaterial in der Lage ist, Leukozyten von dem ersten Durchlaufabschnitt des Urins, der durch dieses durchströmt, zu entfernen.

18. Verfahren zum Erhalt einer Probe vom ersten Durchlauf von Urin eines Spenders, umfassend die Bereitstellung einer Vorrichtung nach einem der Ansprüche 1 bis 17 an einen Spender, wobei der Spender Urin in den ersten Teil der Vorrichtung entleert, und die Sammlung der Probe des ersten Durchlaufs des Urins im zweiten Teil der Vorrichtung.

## Revendications

1. Dispositif pour collecter un échantillon de la partie de premier passage de l'urine vidée par un donneur isolée de la partie de mi-parcours de l'urine, lequel dispositif comporte une première partie (1) comprenant une ouverture à son extrémité supérieure (4) adaptée pour recevoir l'urine vidée par un donneur, un corps de collecte d'urine (3) en aval de l'extrémité supérieure et une sortie fermable (6) située en aval du corps de collecte d'urine et à l'extrémité inférieure (5) de la première partie (1), la sortie (6) fournissant un orifice circulaire (7), une seconde partie (2) située en aval de la sortie (6) de la première partie (1) et comprenant un récipient de collecte qui est en communication avec la sortie (6) de la première partie (1) où l'orifice circulaire fourni par la sortie (6) de la première partie (1) définit une entrée dans le récipient de collecte et a un diamètre qui est plus petit que le diamètre du récipient de collecte à son extrémité supérieure, où le récipient de collecte se présente sous la forme d'un récipient (10) ayant une extrémité fermée (11), lequel récipient contient une bille sphérique (13) ayant un diamètre qui est supérieur au diamètre de l'orifice circulaire (7) pour bloquer l'entrée dans le récipient de collecte, **caractérisé en ce que** la bille sphérique (13) est librement mobile dans le récipient (10) et est capable de flotter dans l'urine de telle manière que, lors de l'utilisation, elle bloque l'entrée dans le récipient de collecte lors du remplissage du récipient de collecte avec l'urine de sorte à entraîner plus d'urine vidée à être collectée dans le corps de collecte d'urine de la première partie et en outre de telle manière que, lors de l'utilisation, la bille (13) est capable de flotter à l'extrémité fermée du récipient (10) lorsque le récipient (10) entre en rotation.

2. Dispositif selon la revendication 1, dans lequel la première partie (1) comprend un entonnoir (3) ayant une sortie fermable (6) fournie au niveau de son extrémité la plus étroite (5).

3. Dispositif selon la revendication 2 qui est logé dans un logement rigide (35) ayant un sommet ouvert fermable (37), le logement ayant un fond (36) adapté de telle manière que le dispositif est capable de reposer de manière stable sur une surface horizontale et plate.

4. Dispositif selon la revendication 3, dans lequel l'entonnoir (3) de la première partie (1) est formé intégralement avec les parois (34) du logement.

5. Dispositif selon la revendication 3 ou la revendication 4, dans lequel l'accès au récipient de collecte dans la seconde partie (2) est fourni par une ouverture (42) dans une paroi latérale du logement (35) de telle manière que, lors de l'utilisation, le contenu du récipient de collecte peut être enlevé du récipient de collecte par l'ouverture (42) dans la paroi latérale du logement (35).

6. Dispositif selon la revendication 5, dans lequel le récipient de collecte est connecté à la paroi latérale du logement (35) au niveau de l'ouverture (42) par l'intermédiaire d'un tube (40) et où la paroi du récipient de collecte séparant le récipient de collecte de l'intérieur du tube comprend une membrane perçable (43).

7. Dispositif selon la revendication 6, dans lequel le tube (40) est formé intégralement dans la paroi du récipient de collecte.

8. Dispositif selon la revendication 6 ou la revendication 7, dans lequel le tube (40) est formé intégralement dans la paroi latérale du logement (35).

9. Dispositif selon l'une quelconque des revendications 6 à 8, dans lequel l'ouverture (42) dans la paroi latérale du logement (35) est fermée par un élément de fermeture ouvrable (44).

10. Dispositif selon l'une quelconque des revendications 3 à 9, dans lequel le sommet ouvert fermable (37) du logement (35) est fourni avec un couvercle (38).

11. Dispositif selon la revendication 10, dans lequel le couvercle (38), sur sa surface interne, a un bouchon se projetant vers le bas (39) moyennant quoi la fermeture du couvercle (38) au sommet (37) du logement (35) entraîne le bouchon (39) à boucher la sortie (27) fournie à l'extrémité inférieure de la première partie.

12. Dispositif selon la revendication 10 ou la revendication 11, dans lequel le couvercle (38) comprend une section formée par une membrane perçable.

13. Dispositif selon la revendication 10 ou la revendication 11, dans lequel le couvercle (38) a une ouverture scellable à l'intérieur.

14. Dispositif selon la revendication 13, dans lequel l'ouverture scellable dans le couvercle (38) est formée pour fournir un embout.

15. Dispositif selon la revendication 1 ou la revendication 2, dans lequel la seconde partie (2) du dispositif est capable d'être détachée de la première partie du dispositif (1).

16. Dispositif selon l'une quelconque des revendications 1 à 15, qui comprend de plus un matériau de filtre au niveau de l'extrémité inférieure de la première partie.

17. Dispositif selon la revendication 16, dans lequel le matériau de filtre est un matériau capable d'enlever les leucocytes de la partie de premier passage de l'urine qui passe à travers celui-ci.

18. Procédé d'obtention d'un échantillon d'urine de premier passage d'un donneur qui comprend la fourniture d'un dispositif selon l'une quelconque des revendications 1 à 17 à un donneur, le donneur vidant l'urine dans la première partie du dispositif, et la collecte de l'échantillon de l'urine de premier passage étant réalisée dans la seconde partie du dispositif.
